# EUROPEAN PATENT APPLICATION

(11) **EP 3 524 222 A1**
(43) Date of publication of application: **14.08.2019**
(21) Application number: 18770714.6
(22) Date of filing: 23.02.2018
(51) Int. Cl.: A61H 39/02, A61B 5/00

(54) **DEVICE AND METHOD FOR THREE-DIMENSIONALLY MAPPING ACUPUNCTURE POINTS**

(30) Priority: 21.03.2017 KR 20170035072
(71) Applicant: Yim, Yun Kyoung, Daejeon 35407 (KR)
(72) Inventor: Yim, Yun Kyoung, Daejeon 35407 (KR)
(74) Representative: Zardi, Marco
(86) International application number: PCT/KR2018/002291
(87) International publication number: WO 2018/174419

(57) **Abstract**

The present invention relates to a three-dimensional acupoint mapping method including the steps of: (a) making use of a three-dimensional standard image inclusive of a three-dimensional image for an internal tissue of the human body to produce a standard template of the internal tissue for determining a standard location of an acupoint; and (b) determining the standard location of the acupoint where a location of the internal tissue is considered on the three-dimensional standard image on the basis of the standard template of the internal tissue to map the determined standard location onto the three-dimensional standard image.

## Description

### Field of the Invention

The present invention relates to a three-dimensional acupoint mapping device and method.

### Background of the Related Art

Acupoints are located on the meridians of the human body to perform acupuncture or moxibustion thereon in traditional eastern medicine, and many technologies for providing the locations of acupoints have been known in conventional practices.

However, the conventional technologies only provide the superficial locations of acupoints on the body surface or the internal locations of acupoints on the basis of a three-dimensional sample made to computer graphics, and accordingly, they do not provide accurate three-dimensional acupoint locations or meridian pathways throughout the human body. Besides, they do not provide accurate information on the safe direction and depth of needle insertion in acupuncture on the basis of the anatomical structures around the acupoint and the acupuncture skill level of an acupuncture practitioner.

When the conventional technologies determine or provide the locations of acupoints, further, they depend upon the self-determination of the practitioner or traditional literatures on acupuncture to provide the locations of acupoints on the surface of a prefabricated fixed sample, and accordingly, they do not consider differences between the anatomical states of individual patients and differences between the acupuncture skill levels of practitioners, thereby unfortunately failing to provide three-dimensional acupoint locations customized to the individual patients and safe acupuncture information customized to the acupuncture skill levels of practitioners.

In addition, most of conventional diagnostic devices of meridian and acupoint are based on physiological characteristics of the acupoints on the body surface, and accordingly, they do not reflect biometric information on acupoints in the deep body.

### Detailed Explanation of the Invention

### Technical Problems

Accordingly, the present invention has been made in view of the above-mentioned problems occurring in the prior art, and it is an object of the present invention to provide a three-dimensional acupoint mapping device and method that provides three-dimensional acupoint locations or meridian pathways in the deep body on the basis of real human body images and provides accurate information on safe direction and depth of needle insertion in acupuncture on the basis of the anatomical structures in the human body.

It is another object of the present invention to provide a three-dimensional acupoint mapping device and method that considers differences between the anatomical states of individual patients and differences between the acupuncture skill levels of practitioners to provide three-dimensional acupoint locations customized to the individual patients and safe acupuncture information customized to the acupuncture skill levels of practitioners.

It is yet another object of the present invention to provide a three-dimensional acupoint mapping device and method that provides three-dimensional meridian and acupoint information to develop a diagnostic device of meridian and acupoint using various biometric information (blood flow speed, the number of blood cells, ion concentration, concentration of specific protein, and so on) in the deep body as well as on the body surface around an acupoint.

It is still another object of the present invention to provide a three-dimensional acupoint mapping device and method that provides three-dimensional meridian and acupoint information to develop an artificial intelligence robot for application of acupuncture, acupressure, meridian massage, and so on.

### Technical Solutions

To accomplish the above-mentioned objects, according to a first aspect of the present invention, there is provided a three-dimensional acupoint mapping method including the steps of:

(a) making use of a standard three-dimensional image inclusive of a three-dimensional image for an internal tissue of the human body to produce a standard template of the internal tissue for determining a standard location of an acupoint; and (b) determining the standard location of the acupoint where a location of the internal tissue is considered on the standard three-dimensional image on the basis of the standard template of the internal tissue to map the determined standard location of the acupoint onto the standard three-dimensional image.

According to the present invention, desirably, the standard three-dimensional image is produced on the basis of a human body image that is obtained by computed tomography (CT), magnetic resonance imaging (MRI), and so on and includes the information on the internal tissue of the human body, and at the step (b), the standard template of the internal tissue on the three-dimensional standard image is produced.

According to the present invention, desirably, at the step (b), the standard location of the acupoint is determined in consideration of the state of at least one of a blood vessel, nerve, muscle, skeleton and organ as the internal tissue.

According to the present invention, desirably, the standard location includes information on direction and/or depth of needle insertion into the acupoint around which the state of the internal tissue is considered.

According to the present invention, desirably, the three-dimensional acupoint mapping method further includes the step of (c) displaying the three-dimensional information of an acpupoint produced by mapping the standard location onto the three-dimensional standard image.

According to the present invention, desirably, the three-dimensional acupoint mapping method further includes the steps of: (d) if a three-dimensional image of a user (patient) is obtained, identifying the internal tissue from the user's three-dimensional image on the basis of the standard template; (e) through the comparison between the user's three-dimensional image and the standard three-dimensional image, calculating a difference between the internal tissue of the user's three-dimensional image and the internal tissue of the standard three-dimensional image; and (f) correcting the standard location on the basis of the calculated difference between the two internal tissues.

According to the present invention, desirably, at the step (f), the standard locations are corrected according to the state of the internal tissue on the user's three-dimensional image on the basis of the difference between the states of the two internal tissues, and through the correction, user-customized three-dimensional acupoint information in the body is produced.

According to the present invention, desirably, at the step (f), the correction on the acupuncture information is performed in consideration of the constitution and type of disease of the user (patient), the acupuncture skill level of the user (practitioner), and the precision of an acupuncture robot, and through the correction, user-customized acupuncture information is produced.

According to the present invention, desirably, at the step (c), if it is determined that the user's input for any one of the three-dimensional acupoint information is generated, detailed acupuncture information on the acupoint corresponding to the user's input is displayed, and the detailed acupuncture information includes numerical information on the direction and/or depth of needle insertion into the acupoint from the body surface to the deep body and information on the internal tissue around the acupoint.

According to the present invention, desirably, at the step (c), at least one operation of enlargement, reduction and rotation operations for the three-dimensional image is performed in response to the user's input related to image control, and through the operation, the information on the internal tissue around the acupoint corresponding to the user's input is displayed according to the depths from the body surface to the deep body.

According to the present invention, desirably, at the step (c), an emphasizing option menu for emphasizing at least one element of the internal tissues on the three-dimensional standard image is displayed, and if the user's input for the emphasizing option menu is generated, the emphasizing option element corresponding to the user's input is identified, so that the identified element is emphasized and displayed on the three-dimensional standard image.

According to the present invention, desirably, at the step (c), three-dimensional meridian pathway information on the body surface and/or in the body is further displayed.

To accomplish the above-mentioned objects, according to a second aspect of the present invention, there is provided a three-dimensional acupoint mapping device including: a standard template producing unit for making use of a three-dimensional standard image inclusive of a three-dimensional image of an internal tissue of the human body to produce a standard template of the internal tissue for determining a standard location of an acupoint; and a mapping unit for determining the standard location of the acupoint where a location of the internal tissue is considered on the three-dimensional standard image on the basis of the standard template of the internal tissue to map the determined standard location onto the three-dimensional standard image, wherein the standard location is three-dimensional coordinates in the body for the acupoint.

According to the present invention, desirably, the three-dimensional standard image is produced on the basis of a human body image inclusive of the information on the internal tissue of the human body, and the standard template producing unit produces the standard template of the internal tissue on the three-dimensional standard image.

According to the present invention, desirably, the mapping unit determines the standard location of the acupoint in consideration of the state of at least one of a blood vessel, nerve, muscle, skeleton and organ as the internal tissue.

According to the present invention, desirably, the standard location includes information on direction and/or depth of needle insertion into the acupoint around which the state of the internal tissue is considered.

According to the present invention, desirably, the three-dimensional acupoint mapping device further includes a display controlling unit for displaying the three-dimensional acupoint information produced by mapping the standard location onto the three-dimensional standard image.

According to the present invention, desirably, the three-dimensional acupoint mapping device further includes: an identification unit for identifying, if a three-dimensional image of a user is obtained, the internal tissue from the user's three-dimensional image on the basis of the standard template; a calculation unit for calculating, through the comparison between the user's three-dimensional image and the standard three-dimensional image, a difference between the internal tissue of the user's three-dimensional image and the internal tissue of the standard three-dimensional image; and a correction unit for correcting the standard location on the basis of the calculated difference between the two internal tissues.

According to the present invention, desirably, the correction unit corrects the standard location according to the state of the internal tissue on the user's three-dimensional image on the basis of the difference between the states of the two internal tissues, and through the correction, user-customized three-dimensional acupoint information in the body is produced.

To accomplish the above-mentioned objects, according to a third aspect of the present invention, there is provided a computer-readable recording medium for recording a program that executes the three-dimensional acupoint mapping method on a computer.

### Advantageous Effects

As described above, the three-dimensional acupoint mapping device and method according to the present invention maps the standard locations of acupoints with the three-dimensional coordinates onto the three-dimensional standard images, thereby providing three-dimensional acupoint locations and meridian pathways in the deep body as well as on the body surface.

Further, the three-dimensional acupoint mapping device and method according to the present invention determines the standard locations of acupoints in consideration of at least one of anatomical structures such as blood vessels, nerves, muscles, and organs of the human body to provide acupuncture information on safe direction and depth of needle insertion on the basis of the locations of the anatomical structures and the acupuncture skill level of the practitioner.

Furthermore, the three-dimensional acupoint mapping device and method according to the present invention utilizes the three-dimensional acupoint information obtained by mapping the three-dimensional coordinates of the acupoints onto the three-dimensional standard image produced on the basis of the human body image, as materials for education and training.

Also, the three-dimensional acupoint mapping device and method according to the present invention emphasizes at least one of the anatomical structures on the basis of the user's input for the emphasizing option menu, so that the real acupuncture can be more safely performed, without any damage on the important anatomical structures such as the blood vessels, organs and nerves around the acupoint.

In addition, the three-dimensional acupoint mapping device and method according to the present invention provides the three-dimensional acupoint locations customized to the patient on the basis of the patient's three-dimensional image, thereby performing the acupuncture according to the patient's characteristics.

Additionally, the three-dimensional acupoint mapping device and method according to the present invention makes use of the three-dimensional acupoint coordinates on the body surface and in the body determined in consideration of the states (locations, sizes, and so on) of the anatomical structure, thereby developing a meridian and acupoint diagnostic device capable of easily analyzing various biometric information (blood flow speed, the number of blood cells, ion concentration, concentration of specific protein, and so on) in the deep body as well as on the body surface around acupoints.

Moreover, the three-dimensional acupoint mapping device and method according to the present invention provides the three-dimensional meridian and/or acupoint information customized to user (patient), thereby allowing an artificial intelligence device (robot) to automatically perform diagnostic analysis on meridian and/or acupoint for the user (patient) and perform safe acupuncture for the user (patient).

Also, the three-dimensional acupoint mapping device and method according to the present invention develops a meridian massage device and an acupressure device based on the three-dimensional information on meridians and acupoints, thereby stimulating more accurately the meridians and acupoints of the user (patient).

### Brief Description of the Drawings

FIG.1 is a block diagram showing a schematic configuration of a three-dimensional acupoint mapping device according to the present invention;
FIG.2 is a view showing a standard three-dimensional image and a user-customized three-dimensional image, which are displayed by the three-dimensional acupoint mapping device according to the present invention;
FIG.3 is a view showing a standard three-dimensional image by acupuncture skill level and a user-customized three-dimensional image by acupuncture skill level, which are displayed by the three-dimensional acupoint mapping device according to the present invention;
FIG.4 is a view showing a standard three-dimensional image by disease type and a user-customized three-dimensional image by disease type, which are displayed by the three-dimensional acupoint mapping device according to the present invention;
FIGS.5a and 5b are views showing an example of a first image displayed by the three-dimensional acupoint mapping device according to the present invention;
FIG.6 is a view showing an example of a second image displayed by the three-dimensional acupoint mapping device according to the present invention;
FIGS.7a and 7b are views showing an example of a third image displayed by the three-dimensional acupoint mapping device according to the present invention;
FIGS.8a to 8c are views showing an example of a fourth image displayed by the three-dimensional acupoint mapping device according to the present invention;
FIG.9 is a view showing an example of determining the location of GB20 by the three-dimensional acupoint mapping device according to the present invention;
FIG.10 is a view showing an example of determining the location of BL40 by the three-dimensional acupoint mapping device according to the present invention;
FIG.11 is a view showing an example of determining the location of GB25 by the three-dimensional acupoint mapping device according to the present invention;
FIG.12 is a view showing an example of determining the location of CV12 by the three-dimensional acupoint mapping device according to the present invention; and
FIG.13 is a flowchart showing a three-dimensional acupoint mapping method according to the present invention.

### -Explanation on Reference Numerals in the Drawing-

100: three-dimensional acupoint mapping device
110: standard template producing unit, 120: mapping unit
130: display controlling unit
140: identification unit, 150: calculation unit
160: correction unit

### DETAILED DESCRIPTION OF THE PREFERRED EMBODIMENTS

Now, an explanation on a three-dimensional acupoint mapping device and method according to the present invention will be given with reference to the attached drawings. Before the present invention is disclosed and described, it is to be understood that the disclosed embodiments are merely exemplary of the invention, which can be embodied in various forms. Therefore, specific structural and functional details disclosed herein are not to be interpreted as limiting.

When real acupuncture is performed on specific acupoints located close to major organs of the human body, much attention is paid to avoid damages of the major organs of the human body, and so as to perform safe acupuncture, accordingly, the direction and depth of needle insertion should be determined in consideration of the tissues (blood vessels, organs, nerves, and muscles as anatomical structures) in the human body and the acupuncture skill level of a practitioner. So as to prevent the principal tissues of the human body from being damaged due to a wrong acupuncture and thus to perform the acupuncture in a more safe manner, the present invention relates to a technology that determines three-dimensional locations of acupoints in consideration of the locations of anatomical structures of the human body to display the location of the determined acupoint in association with a three-dimensional image and that also provides the information for safe acupuncture in consideration of the acupuncture skill level of a practitioner and the precision of an acupuncture robot.

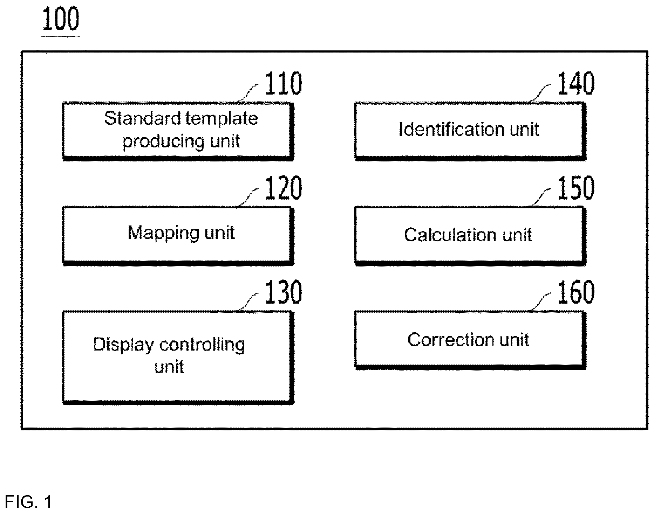

FIG.1 is a block diagram showing a schematic configuration of a three-dimensional acupoint mapping device according to the present invention.

A three-dimensional acupoint mapping device according to the present invention is provided as a type of a computing device having some elements as will be discussed later.

If a device fetches stored commands to sequentially perform the commands through a processor, the device is not limited in a specific type of hardware. The device is provided as a type of a smartphone, a tablet PC, a desktop PC, or the like, thereby being performed in a standalone manner, and otherwise, the device is provided as a type of a server, thereby transmitting processed results to remote clients connected thereto through network.

The standard template producing unit 110 produces a standard template for determining a standard location of an acupoint through a three-dimensional standard image inclusive of at least a given area of the human body.

The three-dimensional standard image is produced on the basis of human body images (or medical images of human body) inclusive of the information on anatomical structures of the human body. In more detail, the three-dimensional acupoint mapping device 100 can reconstruct the human body images inclusive of a plurality of two-dimensional medical images to a three-dimensional image before producing a standard template. In this case, the three-dimensional acupoint mapping device 100 performs the reconstruction for the two-dimensional medical images to allow at least one of skin, blood vessels, nerves, muscles, and organs as the anatomical structures of the human body therein to be contained in the three-dimensional image, thereby producing the three-dimensional standard image.

Further, the medical human body images used upon the production of the three-dimensional standard image are obtained by computed tomography (CT), magnetic resonance imaging (MRI), positron emission tomography (PET), and so on, and of course, the three-dimensional standard image may be produced on the basis of the images obtained by other image photographing devices.

Accordingly, information on the anatomical structures such as blood vessels, nerves, tendons, ligaments, muscles, and organs is contained in the three-dimensional standard image, but without being limited thereto, information on all anatomical structures to be considered when three-dimensional coordinates of an acupoint are determined or when acupuncture is performed is contained in the three-dimensional standard image.

Also, the standard template producing unit 110 produces the standard template for an anatomical structure in the three-dimensional standard image. At this time, the standard template produced by the standard template producing unit 110 is used to determine the standard location of an acupoint. If an user (patient)'s three-dimensional image (which is produced by reconstructing the medical human body image of the user obtained by CT, MRI, and so on) is given, further, the standard template is used to identify an anatomical structure in the user's three-dimensional image. For example, the standard template producing unit 110 produces a standard template for at least one of anatomical structures, such as a liver template, a muscle template, a bone template, and so on.

The mapping unit 120 determines the standard locations of the acupoints on the three-dimensional standard image on the basis of the standard template and thus maps the determined standard location onto the three-dimensional standard image. In this case, the standard locations indicate three-dimensional coordinates on the body surface and in the body for the acupoints. That is, the mapping unit 120 provides the three-dimensional acupoint locations (or coordinates) on the body surface and in the body through the three-dimensional standard image.

The three-dimensional information of an acupoint is produced by the mapping unit 120 and is thus displayed on a screen by means of a display controlling unit 130 as will be discussed later. In this case, the three-dimensional acupoint information includes the three-dimensional standard image and the information on the standard locations of the acupoints. Further, the display controlling unit 130 displays the three-dimensional acupoint information.

At this time, the mapping unit 120 determines the standard locations (three-dimensional coordinates) of acupoints in consideration of the state of at least one of the anatomical structures such as blood vessels, nerves, muscles, skeleton, and organs. In this case, the mapping unit 120 considers the location or size of the anatomical structure as the state of the anatomical structure. Moreover, the standard locations include information on direction and/or depth of needle insertion for safe acupuncture under the consideration of the state of the anatomical structure. That is, the mapping unit 120 determines the standard locations (three-dimensional coordinates) of the acupoints on the basis of the location and size of the anatomical structure on the three-dimensional standard image, and maps the determined standard locations of the acupoints onto the three-dimensional standard image. The standard locations of all acupoints of the human body are displayed on the three-dimensional standard image, and the information on safe acupuncture is provided according to the acupuncture skill level of a practitioner and the precision of an acupuncture robot.

On the other hand, the three-dimensional acupoint mapping device 100 according to the present invention includes the display controlling unit 130 for displaying the three-dimensional acupoint information produced by mapping the standard locations of the acupoints onto the three-dimensional standard image on a screen.

Also, the three-dimensional standard image with / without the standard location of at least one acupoint is displayed on the screen by means of the display controlling unit 130. Furthermore, the display controlling unit 130 displays three-dimensional standard image with/without the standard three-dimensional pathway of at least one meridian on the body surface and/or in the body on the screen. Accordingly, the display controlling unit 130 displays information on the distribution of meridians or locations of acupoints from the body surface to the deep inside the body. Also, the display controlling unit 130 displays the information on the acupoint locations and the meridian pathways on the body surface and/or in the deep body in association with a three-dimensional image of the user (patient) as will be discussed later.

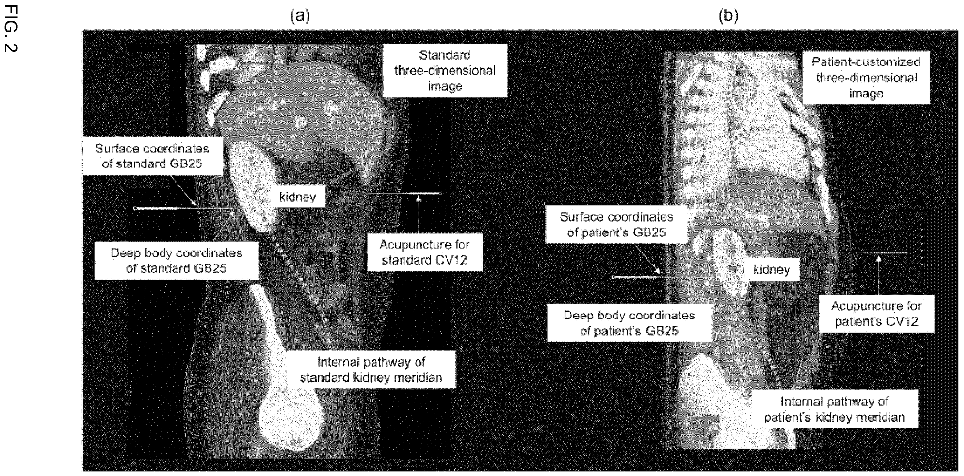

For example, the kidney meridian passes through the kidneys, liver, lungs and heart in the body. As shown in FIG.2, the three-dimensional acupoint mapping device 100 according to the present invention maps the information on the pathway of the kidney meridian in association with the kidney, liver, lung and heart in the body onto the three-dimensional standard image or the three-dimensional image of the user (patient) and then displays the mapped information. At this time, the three-dimensional acupoint mapping device 100 according to the present invention displays any one of the three-dimensional standard image or the three-dimensional image of the user (patient), and if necessary, displays both of the three-dimensional standard image and the three-dimensional image of the user (patient).

If it is determined that the input of the user for any one of the displayed three-dimensional acupoint in the body is generated, the display controlling unit 130 displays detailed acupuncture information on the acupoint corresponding to the user's input. In this case, the detailed acupuncture information includes an acupuncture needle icon placed on the acupoint corresponding to the user's input. Further, the detailed acupuncture information includes numerical information on the direction and/or depth of needle insertion into the acupoint corresponding to the user's input. Furthermore, the detailed acupuncture information includes information on the anatomical structure around the acupoint corresponding to the user's input. In relation to the detailed acupuncture information, that is, the display controlling unit 130 displays the acupuncture needle icon placed on the acupoint corresponding to the user's input, displays the information on the direction and/or depth of needle insertion from the surface coordinates of the acupoint to the deep body coordinates of the acupoint corresponding to the user's input, or displays the information on the identification names of the anatomical structures around the acupoint corresponding to the user's input. In this case, the information on the depth of needle insertion indicates depth information from the body surface to a point where the end of a needle reaches, and the information on the direction of needle insertion indicates information on the direction of the needle with respect to the body surface.

Further, the display controlling unit 130 performs at least one of enlargement, reduction and rotation operations for the three-dimensional image in response to an input (for example, drag, separate menu click, or the like) of the user related to image control. At this time, the user's input related to the image control to perform the enlargement, reduction and rotation operations for the three-dimensional image is carried out through previously set drag-and-drop operations in correspondence to respective functions of the image control or through click operations on the icons corresponding to the respective functions of the image control.

Through the operations related to the image control, the display controlling unit 130 displays the information on the anatomical structure around the acupoint corresponding to the user's input according to the depths from the body surface to the deep body. That is, the display controlling unit 130 displays the three-dimensional image on which the anatomical structure (for example, blood vessel, nerve, muscle, skeleton, and organ) around the acupoint corresponding to the user's input is projected according to the depths from the body surface to the deep body. Particularly, the display controlling unit 130 displays the anatomical structure around the needle inserted into a given acupoint through the three-dimensional image varied according to various depths and angles. Accordingly, the three-dimensional acupoint mapping device 100 according to the present invention provides accurate information on the three-dimensional coordinates in the body for the acupoint so that safe acupuncture can be performed according to the acupuncture skill level of the practitioner and the precision of the acupuncture robot.

In addition, the display controlling unit 130 displays an emphasizing option menu for emphasizing at least one of the anatomical structures in the three-dimensional image. If the user's input for the emphasizing option menu is generated, at this time, the display controlling unit 130 identifies an element for an emphasizing option corresponding to the user's input and emphasizes and displays the identified element on the three-dimensional image. At this time, examples of the element for the emphasizing option include blood vessels, nerves, muscles, skeleton, and organs, but they are not limited thereto. Through the emphasizing option menu, real acupuncture for the given acupoint can be performed more safely.

For example, the display controlling unit 130 displays 'blood vessel emphasizing option menu' for determining whether a blood vessel among the anatomical structures on the three-dimensional image is emphasized or not (YES/NO) on the screen. In this case, if it is determined that the blood vessel is emphasized (YES) by the user's input for the 'blood vessel emphasizing option menu', the display controlling unit 130 emphasizes and displays the portion corresponding to the blood vessel on the three-dimensional image displayed on the screen so that the portion is vividly provided for the eyes of the user.

The emphasis on the anatomical structures on the three-dimensional standard image is performed on the basis of the user's input, but it is not limited thereto. On the basis of the information on previously inputted structures with respect to specific acupoints, that is, the previously inputted structures can be automatically emphasized. In this case, the information on the previously inputted structures with respect to the specific acupoints indicates information on the structures to which special attention is paid when the real acupuncture for the specific acupoints is performed.

For example, if a first acupoint is located close to an organ to cause much attention to be paid to the real acupuncture for the first acupoint so as to prevent the organ from being touched by the needle, matching information of the location (three-dimensional coordinates in the body) of the first acupoint with the organ is in advance stored in the three-dimensional acupoint mapping device 100. If the user's input for the first acupoint is generated, after that, the display controlling unit 130 automatically emphasizes and displays the organ on the three-dimensional standard image on the screen.

Through the emphasis and display of the anatomical structure, the three-dimensional acupoint mapping device 100 according to the present invention can perform the real acupuncture for the given acupoint more safely. That is, the three-dimensional acupoint mapping device 100 according to the present invention can emphasize and display at least one (for example, blood vessel, nerve, muscle, skeleton, organ, tendon, ligament, and so on) of the anatomical structures on the three-dimensional image, so that the real acupuncture can be more safely performed, without any damage on the blood vessels, organs and nerves of the user (patient)'s body.

So as to provide user-customized three-dimensional information on meridians and acupoints, on the other hand, the three-dimensional acupoint mapping device 100 according to the present invention includes an identification unit 140, a calculation unit 150, and a correction unit 160.

If the user (patient)'s three-dimensional image is obtained, the identification unit 140 identifies the anatomical structure on the user's three-dimensional image on the basis of the standard template. In this case, the user's three-dimensional image is an image produced by three-dimensionally reconstructing the body image of the user obtained by MRI, CT, and so on. For example, the identification unit 140 identifies the liver from the user's three-dimensional image on the basis of a standard liver template and the kidney from the user's three-dimensional image on the basis of a standard kidney template.

Further, the identification unit 140 performs a normalization process of matching the user's three-dimensional image with the standard image so as to allow the anatomical structure to be easily identified from the three-dimensional image on the basis of the standard template.

Through the comparison between the user's three-dimensional image and the standard three-dimensional image, the calculation unit 150 calculates a difference between the anatomical structure of the user's three-dimensional image and the anatomical structure of the standard three-dimensional image.

The correction unit 160 corrects the standard location on the basis of the calculated difference between the two anatomical structures. On the basis of the difference between the states of the two anatomical structures, that is, the correction unit 160 corrects the standard location set with respect to the three-dimensional standard image to the standard location set with respect to the state of the anatomical structure on the user's three-dimensional image. In this case, the anatomical structure states include the location and size of the anatomical structure. Through the correction of the correction unit 160, accordingly, the user-customized three-dimensional acupoint information in the body can be produced.

In more detail, individual users (patients) have different locations and sizes of their anatomical structure, and so as to provide the locations of the acupoints customized to the individual users, accordingly, there is a need to correct the standard location set with respect to the standard three-dimensional image in consideration of the location and size of the user's anatomical structure.

On the three-dimensional standard image, for example, if it is assumed that the surface of the liver is located at a point corresponding to a first distance (for example, 50 mm) from the body surface and the standard location of the first acupoint is placed at a point (corresponding to a distance of 48 mm from the body surface) corresponding to a distance of 2 mm from the surface of the liver within the first distance, it is found that the user's liver is more swollen than the liver on the three-dimensional standard image through the comparison between the user's three-dimensional image and the standard three-dimensional image by means of the calculation unit 150, and accordingly, it is assumed that the distance from the body surface of the user (patient) to the surface of the liver of the user has a smaller value (for example, 45 mm) than a distance obtained by subtracting the distance of 2 mm from the first distance. At this time, if the real acupuncture for the user is performed on the basis of the standard location of the first acupoint, the needle is inserted into the user's liver because the user (patient)'s liver is swollen, so that the user's liver may be damaged. So as to perform safe acupuncture, accordingly, the three-dimensional acupoint mapping device 100 according to the present invention can correct the coordinates in the deep body for the first acupoint on the basis of the state of the anatomical structure, that is, the state (location and size) of the liver on the user's three-dimensional image by means of the correction unit 160. At this time, for example, the coordinates in the deep body for the first acupoint corrected by means of the correction unit 160 are located at a point corresponding to the distance of 2 mm from the user (patient)'s liver so as to prevent the user (patient)'s liver from being inserted by the needle. The corrected coordinates in the deep body for the first acupoint are used as the user-customized safe acupuncture depth information.

In addition, for example, if it is inappropriate to insert the needle due to obstacles (for example, damages, lesions, foreign bodies, and so on) on the body surface coordinates or between the body surface coordinates and the coordinates in the deep body for the user (patient)'s first acupoint, the body surface coordinates for the first acupoint can be corrected by means of the correction unit 160 to perform safe acupuncture. The corrected body surface coordinates for the first acupoint are used as the user-customized safe acupuncture direction information.

If the user's input for correction of the acupuncture information of the acupoint is generated, the correction unit 160 performs the correction on the safe acupuncture information in consideration of the acupuncture skill level of the practitioner and the precision of the acupuncture robot. At this time, an amount of correction according to the acupuncture skill level of the practitioner and the precision of the acupuncture robot is in advance stored in the three-dimensional acupoint mapping device 100. For example, if the acupuncture skill level of the practitioner is inputted as a beginning level, the safe acupuncture information is corrected by a first value, and if the acupuncture skill level of the practitioner is inputted as an intermediate level, the safe acupuncture information is corrected by a second value.

As adequate acupuncture methods (directions, depths, speeds, and so on) are different according to diseases, the correction unit 160 performs the correction on the acupuncture information on the acupoint in consideration of the kinds of the patient's diseases. At this time, an amount of correction according to the diseases is in advance stored in the three-dimensional acupoint mapping device 100. For example, if disease A as the patient's disease is inputted, the acupuncture information (depth of needle insertion, direction of needle insertion, speed of needle insertion, and so on) on the first acupoint is corrected by a value of a, and if disease B as the patient's disease is inputted, the acupuncture information on the first acupoint is corrected by a value of b.

At this time, the user-customized three-dimensional acupoint and acupuncture information produced by means of the correction unit 160 is displayed on the screen through the display controlling unit 130 in the same manner as the three-dimensional acupoint information in the body based on the three-dimensional standard image.

Further, the three-dimensional acupoint mapping device 100 according to the present invention performs machine learning through the acupoint location information corrected according to the individual users, and on the basis of the machine learning, the correction unit 160 provides the user-customized acupoint location and acupuncture information more accurately.

Under the above-mentioned configuration of the three-dimensional acupoint mapping device 100, now, an explanation on the examples of the screens on which the three-dimensional acupoint information in the body and/or the user-customized three-dimensional acupoint information in the body produced by the three-dimensional acupoint mapping device 100 are displayed will be given.

FIG.2 shows the examples of the standard three-dimensional image and the user (patient)-customized three-dimensional image displayed by the three-dimensional acupoint mapping device 100 according to the present invention.

Referring to (a) of FIG.2, the display controlling unit 130 displays (or provides) the three-dimensional standard image on the screen. At this time, the display controlling unit 130 displays the three-dimensional acupoint information in the body based on the three-dimensional standard image.

For example, the display controlling unit 130 maps and provides the standard location in the deep body (that is, standard coordinates in the deep body) for GB25. Further, the display controlling unit 130 provides the standard location of the body surface (that is, standard coordinates on the body surface) for GB25 in association with the three-dimensional standard image. Furthermore, the display controlling unit 130 displays the acupuncture needle icon located on the corresponding GB25 on the screen on the basis of the standard coordinates on the body surface for GB25 and the standard coordinates in the deep body for GB25. Also, the display controlling unit 130 provides the standard acupuncture information (inclusive of the information on the standard coordinates on the body surface, the standard coordinates in the deep body, standard direction of needle insertion, standard depth of needle insertion, standard speed of needle insertion, and so on) for one acupoint (for example, GB25). In addition, the display controlling unit 130 displays acupuncture information on two or more of acupoints (for example, GB25 and CV12). As mentioned above, further, the display controlling unit 130 displays internal and/or external meridian pathway information inclusive of the three-dimensional coordinate information on the body surface or in the deep body on the three-dimensional standard image. For example, a screen (a) of FIG.2 shows an example where the internal pathway of the kidney meridian flowing in association with the kidney, liver, lung and heart in the body is mapped onto the three-dimensional standard image and displayed.

As shown in a screen (b) of FIG.2, the display controlling unit 130 displays (or provides) the user (patient)'s three-dimensional image on the screen. At this time, the display controlling unit 130 displays the user-customized three-dimensional acupoint information in the body based on the user's three-dimensional image. For example, the display controlling unit 130 maps the coordinates in the deep body for the user's GB25 (that is, the patient's GB25 coordinates in the deep body) determined on the basis of the state of the anatomical structure of the user's three-dimensional image onto the user's three-dimensional image. In this case, the user's GB25 coordinates in the deep body are determined by correcting the standard location of GB25 in the deep body in consideration of the state of the anatomical structure (for example, the location and size of the kidney) of the user's three-dimensional image through the comparison between the standard three-dimensional image and the user's three-dimensional image.

Further, the display controlling unit 130 provides the user-customized GB25 standard coordinates on the body surface (that is, the patient's GB25 coordinates on the body surface) customized to the user in association with the user's three-dimensional image. Further, the display controlling unit 130 displays the acupuncture needle icon located on the corresponding GB25 on the screen on the basis of the user's GB25 coordinates on the body surface and the user's GB25 coordinates in the deep body. Also, the display controlling unit 130 provides the acupuncture information (inclusive of information on the coordinates on the body surface, the coordinates in the deep body, direction of needle insertion, depth of needle insertion, and so on, which are determined in consideration of the state of the anatomical structure in the patient's body) for an acupoint (for example, GB25), as acupuncture information customized to the user. In addition, the display controlling unit 130 displays acupuncture information on two or more of acupoints (for example, GB25 and CV12). For example, the acupuncture needle icon for CV12 displayed on the user's three-dimensional image indicates the acupuncture needle inserted into patient's CV12 determined according to the patient's state. Further, the display controlling unit 130 displays meridian pathway information inclusive of the three-dimensional coordinate information on the body surface and/or in the deep body on the user's three-dimensional image. For example, a screen (b) of FIG.2 shows an example where the internal pathway of the kidney meridian flowing in association with the kidney, liver, lung and heart of the body is mapped onto the user's three-dimensional image and is displayed.

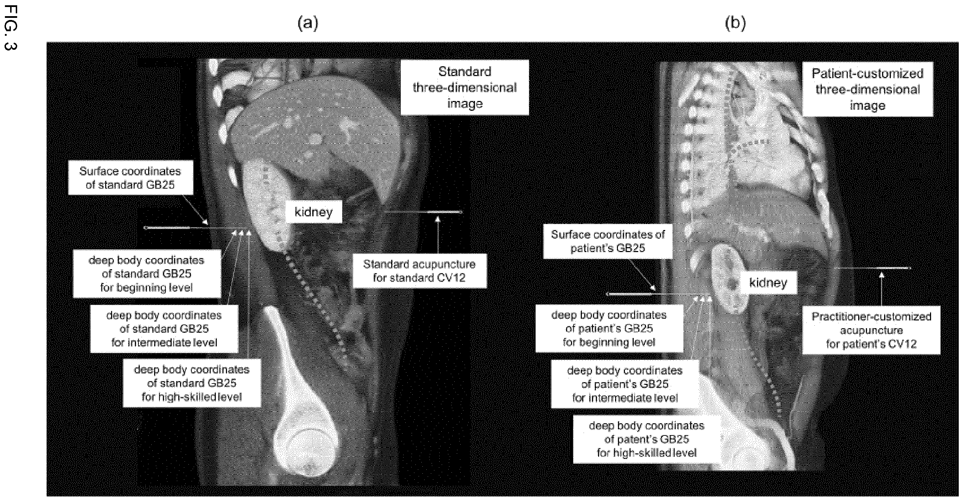

FIG.3 shows the examples of safe acupuncture information by acupuncture skill level of practitioner on the standard three-dimensional image and the user-customized three-dimensional image displayed by the three-dimensional acupoint mapping device 100 according to the present invention. For example, the display controlling unit 130 provides safe acupuncture information for GB25 determined according to the state of the anatomical structure (the location and size of the kidney) and the acupuncture skill level of practitioner (e.g. beginning level, intermediate level, high-skilled level) on the standard three-dimensional image or the user-customized three-dimensional image.

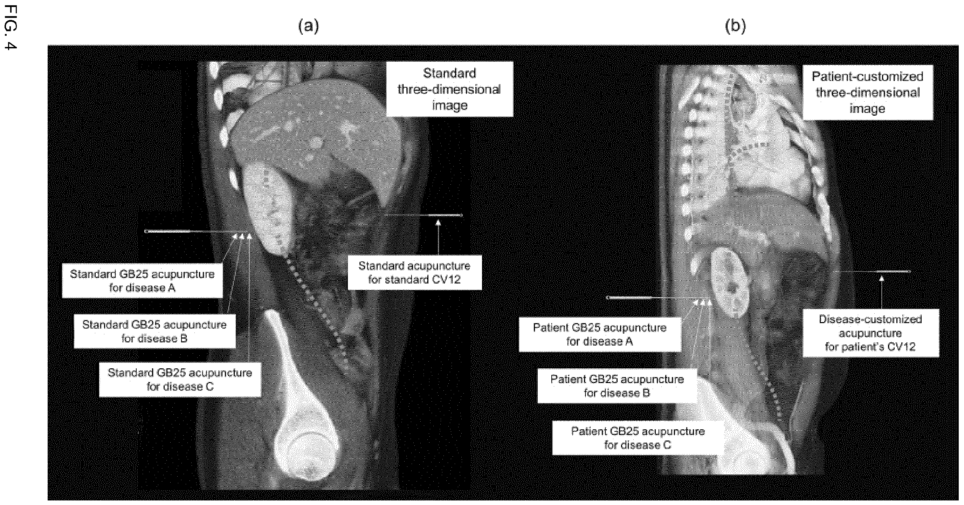

FIG.4 shows the examples of acupuncture information by disease type on the standard three-dimensional image and the user-customized three-dimensional image displayed by the three-dimensional acupoint mapping device 100 according to the present invention. For example, the display controlling unit 130 provides acupuncture information for GB25 determined according to the disease type inputted on the standard three-dimensional image or the user-customized three-dimensional image.

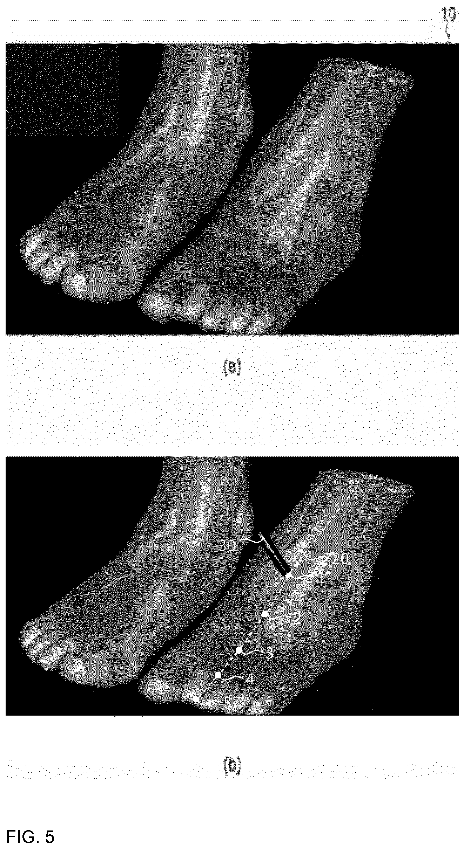

FIGS.5a and 5b are views showing an example of a first image displayed by the three-dimensional acupoint mapping device 100 according to the present invention.

As shown in FIG.5, the display controlling unit 130 displays the three-dimensional image 10 on the screen.

At this time, the three-dimensional image 10 as shown in FIG.5 is the three-dimensional standard image or the user's three-dimensional image. As shown in FIGS.5a and 5b, the three-dimensional image 10 for feet as a portion of the user's body is displayed.

As shown in FIG.5b, the display controlling unit 130 maps a meridian pathway 20 including the location information on first to fifth acupoints 1 to 5 onto the three-dimensional image 10 and displays the mapped result. At this time, the acupoint location information and/or the meridian pathway information provided by the three-dimensional acupoint mapping device 100 according to the present invention are information on the three-dimensional coordinates on the body surface and in the body.

As shown in FIG.5b, the first acupoint 1 indicates the acupoint corresponding to ST41, the second acupoint 2 the acupoint corresponding to ST42, the third acupoint 3 the acupoint corresponding to ST43, the fourth acupoint 4 the acupoint corresponding to ST44, and the fifth acupoint 5 the acupoint corresponding to ST45. If the user's input for the first acupoint 1 among the first to fifth acupoints is generated, as shown in FIG.5b, an acupuncture needle icon 30 inserted into the first acupoint 1 is displayed in association with the three-dimensional image 10.

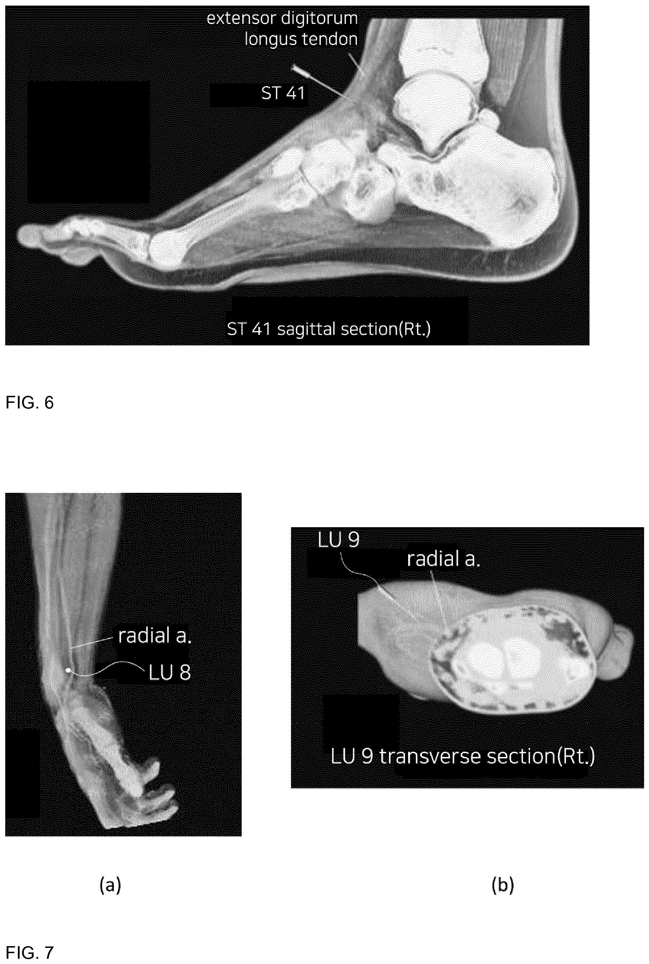

Further, the display controlling unit 130 displays a sectional image of the three-dimensional image for the location of a specific acupoint. In this case, the display controlling unit 130 also displays an acupuncture needle icon inserted into the specific acupoint on the three-dimensional image. Further, the display controlling unit 130 displays images having various sections like sagittal section, transverse section, and so on according to the user's input. An example of the sectional image display is shown in FIG.6.

FIG.6 is a view showing an example of a second image (e.g., sectional image) displayed by the three-dimensional acupoint mapping device 100 according to the present invention.

FIG.6 shows the sagittal sectional image with the needle inserted into the first acupoint 1 (that is, the needle inserted into ST41) as shown in FIG.5b. For example, the location of ST41 (inclusive of the location on the body surface and in the body) and acupuncture on ST41 (inclusive of the information on the depth and direction of needle insertion) are determined as the three-dimensional coordinates in the body with respect to the location of an extensor digitorum logus tendon as an anatomical structure.

FIGS.7a and 7b are views showing an example of a third image displayed by the three-dimensional acupoint mapping device 100 according to the present invention.

As shown in FIGS.7a and 7b, the three-dimensional acupoint mapping device 100 according to the present invention determines the standard locations of LU8 and LU9 with respect to the radial artery. Further, as shown in FIG.7b, the three-dimensional acupoint mapping device 100 according to the present invention displays the determined standard location of LU9 through a sectional image in consideration of the state of the anatomical structure (e.g., the radial artery) in the body.

As shown in FIGS.7a and 7b, further, the three-dimensional acupoint mapping device 100 emphasizes and displays the radial artery on the three-dimensional image so as to prevent the radial artery from being damaged upon the acupuncture. Accordingly, the three-dimensional acupoint mapping device 100 guides safe acupuncture into the corresponding acupoint on the basis of the three-dimensional image on which the blood vessel is emphasized.

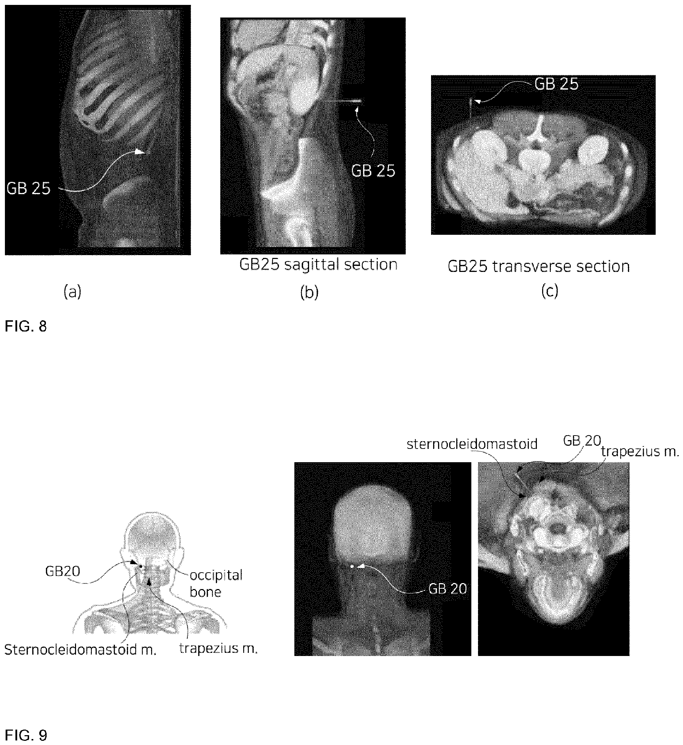

FIGS.8a to 8c are views showing an example of a fourth image displayed by the three-dimensional acupoint mapping device 100 according to the present invention.

FIG.8a shows the acupoint GB25 displayed on a CT image. The acupoint GB25 is located over the kidney, and when acupuncture is performed on GB25, attention should be paid to avoid damaging the kidney. Accordingly, the three-dimensional acupoint mapping device 100 according to the present invention determines the three-dimensional coordinates of GB25 in the deep body in consideration of the location of the kidney.

Referring especially to FIG.8b showing the transverse sectional image of the acupoint GB25 and to FIG.8c showing the sagittal sectional image of the acupoint GB25,
the three-dimensional acupoint mapping device 100 according to the present invention displays the determined acupoint location and the acupuncture needle icon located on the corresponding acupoint in consideration of the location of the anatomical structure in the body on the three-dimensional image, so that the accurate location of the kidney under the acupoint GB25 is provided for the user, and without any damage on the kidney upon the acupuncture, accordingly, the needling depth and direction for safe acupuncture are three-dimensionally provided for the user.

FIG.9 is a view showing an example of determining the standard location of GB20 by the three-dimensional acupoint mapping device 100 according to the present invention.

As shown in FIG.9, GB20 is located in the anterior region of the neck, inferior to the occipital bone, in the depression between the origins of sternocleidomastoid muscle and the trapezius muscle.

Also, GB20 is used to treat various diseases such as stroke, eye pain, nosebleed, common cold, insomnia, stiff neck, and so on. The depth and direction of needle insertion on this point depend on the type of disease to be treated.

For example, the direction of needle insertion on GB20 includes a perpendicular insertion, an oblique insertion (needle insertion toward the opposite side eyeball), and a transverse insertion (transverse needle insertion toward right side at left GB20 and toward left side at right GB20), and the depth of needle insertion at GB20 depends on diseases to be treated or the condition of the patient.

So as to determine the standard location (that is, the three-dimensional coordinates) of GB20, the three-dimensional acupoint mapping device 100 according to the present invention produces the standard templates of the occipital bone, sternocleidomastoid muscle, and trapezius muscle on the three-dimensional standard image by means of the standard template producing unit 110. After that, the mapping unit 120 determines the three-dimensional X, Y and Z coordinates in the body as the standard location of GB20 on the basis of the occipital bone, sternocleidomastoid muscle, and trapezius muscle as the anatomical structures corresponding to the standard templates.

If the three-dimensional image of the patient is inputted, also, the identification unit 140 of the three-dimensional acupoint mapping device 100 according to the present invention applies the standard templates (that is, the standard occipital bone template, the standard sternocleidomastoid muscle template, and the standard trapezius muscle template) produced on the basis of the standard three-dimensional image to the patient's three-dimensional image. Accordingly, the identification unit 140 identifies the occipital bone, sternocleidomastoid muscle, and trapezius muscle as the anatomical structures of the patient from the three-dimensional image of the patient.

Next, the calculation unit 150 compares the standard three-dimensional image and the patient's three-dimensional image. Otherwise, the calculation unit 150 compares the standard templates and the anatomical structures identified from the patient's three-dimensional image. For example, the standard occipital bone template is compared with the occipital bone identified from the patient's three-dimensional image.

The individual patients have different locations or sizes of their anatomical structure, and accordingly, the calculation unit 150 calculates a difference between the states of the two anatomical structures through the comparison between the two images or between the two anatomical structures. On the basis of the difference between the states of the two anatomical structures, after that, the correction unit 160 corrects the X, Y and Z coordinates as the standard location of GB20 to X', Y' and Z' coordinates according to the states of the anatomical structures of the patient. At this time, the direction and depth of needle insertion depend on diseases, and accordingly, amounts of correction of GB20 are varied according to the diseases of the patient in advance inputted. For example, if a disease A is inputted as the disease information of the patient, the correction unit 160 corrects the coordinates of GB20 to X'a, Y'a and Z'a, and if a disease B is inputted as the disease information of the patient, the correction unit 160 corrects the coordinates of GB20 to X'b, Y'b and Z'b. Like this, the three-dimensional acupoint mapping device 100 according to the present invention provides the individualized and customized acupuncture information based on the disease to be treated.

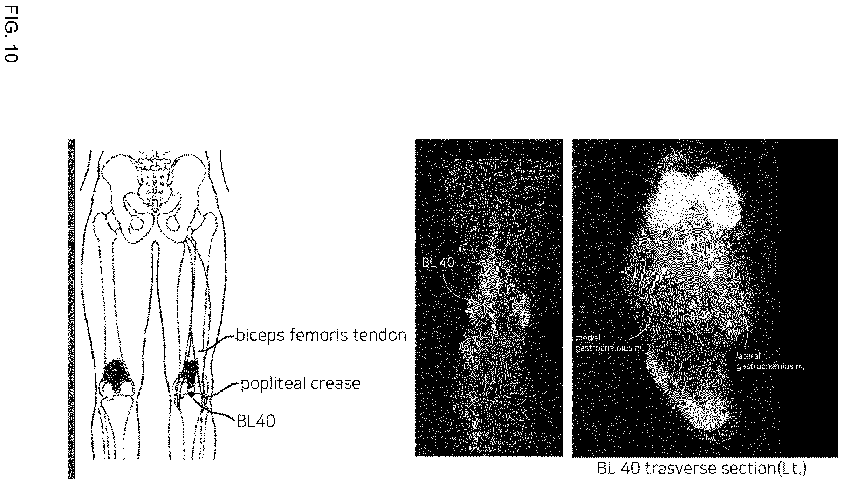

FIG.10 is a view showing an example of determining the location of BL40 by the three-dimensional acupoint mapping device 100 according to the present invention.

BL40 is located on the posterior aspect of the knee, at the midpoint of the popliteal crease between the biceps femoris tendon and the semitendinosus tendon.

Also, BL40 is used to treat various diseases such as lumbago, epistaxis, fever, skin ulcer, difficult urination, and so on. The acupuncture technique for this point varies according to the diseases to be treated and the conditions of the patient.

When acupuncture is performed on BL40, attention should be paid to avoid damaging the popliteal artery or tibial nerve. When a pricking bloodletting technique (that is, a therapeutic method to let out a small amount of blood) is applied to this point, the acupuncture needle pricks the popliteal vein. In this case, the needle is inserted and withdrawn at a rapider speed than a general speed.

To perform acupuncture on BL40, the mapping unit 120 of the three-dimensional acupoint mapping device 100 according to the present invention determines the standard location of BL40 (the standard three-dimensional X, Y and Z coordinates of BL40,) on the basis of the locations of the anatomical structures on the standard three-dimensional image and provides the standard acupuncture information (direction, depth, and speed of needle insertion) so that upon the acupuncture, the needle does not come into contact with the popliteal artery and the tibial nerve.

After that, the identification unit 140 determines the location of patient's BL40 (the patient-customized three-dimensional X', Y' and Z' coordinates) on the basis of the anatomical structures such as biceps femoris tendon and semitendinosus tendon on the patient's three-dimensional image.

Further, the identification unit 140 identifies the popliteal artery from the patient's three-dimensional image with the popliteal artery template produced on the basis of the standard three-dimensional image, and with the identified result, the acupuncture information for patient's BL40 is corrected through the correction unit 160. If the patient's BL40 coordinates X', Y' and Z' come into contact with the popliteal artery identified from the patient's three-dimensional image or pass therethrough, the correction unit 160 corrects the coordinates of patient's BL40 to X'a, Y'a and Z'a on the basis of the location of the popliteal artery as the anatomical structure of the patient, so that upon the acupuncture of BL40, the needle does not prick the popliteal artery.

When a pricking bloodletting technique is applied to BL40, the correction unit 160 corrects the coordinates of patient's BL40 to X'b, Y'b and Z'b so as to allow the needle to come into contact with the popliteal vein, and then corrects the speeds of needle insertion and withdrawal, thereby providing the adequate acupuncture information with which the pricking bloodletting is achieved on BL40. At this time, the display controlling unit 130 emphasizes and displays the popliteal vein as one of the anatomical structures contained in the patient's three-dimensional image. Like this, the three-dimensional acupoint mapping device 100 according to the present invention provides the user-customized acupuncture information based on the treatment purpose and the acupuncture technique.

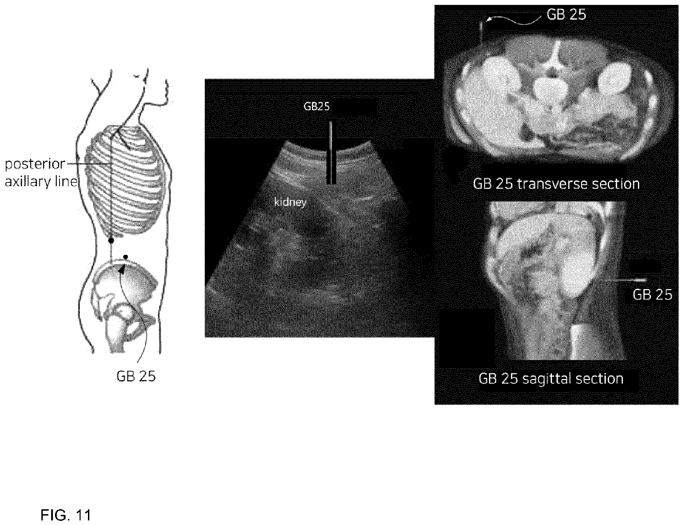

FIG.11 is a view showing an example of determining the location of GB25 by the three-dimensional acupoint mapping device 100 according to the present invention.

As shown in FIG.11, GB25 is located on the lateral abdomen, inferior to the free extremity of the 12th rib. The free extremity of the 12th rib can be palpated below the inferior border of the costal arch posterior to the posterior axillary line.

The direction and depth of needle insertion into GB25 depends on the disease to be treated or the condition of the patient. Further, there is the kidney in the deep portion under GB25, and when acupuncture is performed on GB25, accordingly, attention should be paid to avoid damaging the kidney.

The mapping unit 120 determines the standard X, Y and Z coordinates of GB25, on the standard three-dimensional image on the basis of the standard templates for the anatomical structures such as the free extremity of the 12th rib, the kidney, and so on.

If the patient's three-dimensional image is inputted, after that, the calculation unit 150 compares the patient's three-dimensional image with the standard three-dimensional image, and the correction unit 160 corrects the coordinates of GB25 to patient-customized X', Y' and Z' coordinates on the basis of the correction condition in advance set to prevent the kidney from being pricked by the needle in consideration of the anatomical structures of the patient. For example, the correction unit 160 corrects the coordinates of GB25 to the coordinates corresponding to a location distant by a given distance from the surface of the kidney of the patient or a location corresponding to a given percentage of a distance from the surface of the body of the patient to the surface of the kidney of the patient.

Further, the correction unit 160 corrects the safe acupuncture information in consideration of the acupuncture skill level of the practitioner. If the acupuncture practitioner is at a beginning level, for example, the correction unit 160 corrects the coordinates to X'a, Y'a and Z'a corresponding to a location distant by a distance 'a' from the surface of the kidney of the patient or a location corresponding to 70% of a distance from the surface of the body of the patient to the surface of the kidney of the patient, if he or she is at an intermediate level, the correction unit 160 corrects the coordinates to X'b, Y'b and Z'b corresponding to a location distant by a distance 'b' from the surface of the kidney of the patient or a location corresponding to 80% of a distance from the surface of the body of the patient to the surface of the kidney of the patient, and if he or she is at a high skilled level, the correction unit 160 corrects the coordinates to X'c, Y'c and Z'c corresponding to a location distant by a distance 'c' from the surface of the kidney of the patient or a location corresponding to 90% of a distance from the surface of the body of the patient to the surface of the kidney of the patient. Like this, the three-dimensional acupoint mapping device 100 according to the present invention provides the practitioner-customized safe acupuncture information based on the acupuncture skill level of the acupuncture practitioner.

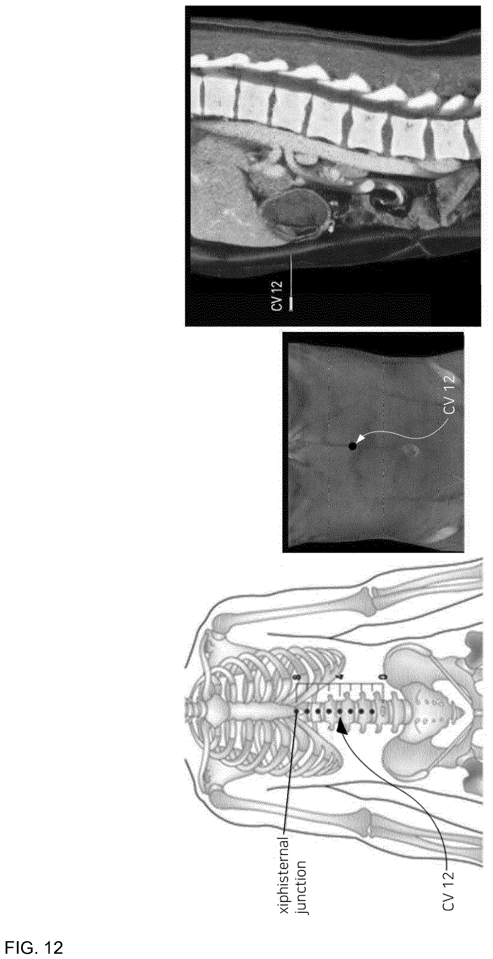

FIG.12 is a view showing an example of determining the location of CV12 by the three-dimensional acupoint mapping device 100 according to the present invention.

As shown in FIG.12, CV12 is located on the upper abdomen, on the anterior median line, at the midpoint of the line connecting the xiphisternal junction and the centre of umbilicus.

Some of patients have the swollen liver or the liver located at a lower place than a general place, and accordingly, the acupuncture on CV12 is performed with carefulness.

The mapping unit 120 determines the standard three-dimensional location of CV12 (standard X, Y and Z coordinates of CV12,) on the standard three-dimensional image on the basis of the xiphisternal junction and the umbilicus.

If the patient's three-dimensional image is inputted, after that, the calculation unit 150 compares the patient's three-dimensional image with the standard three-dimensional image, and the correction unit 160 performs the correction on the location of CV12 according to the disease and the condition of the patient in advance inputted.

The individual patients have different locations of the liver, and the three-dimensional acupoint mapping device 100 according to the present invention identifies the location of the liver from the patient's three-dimensional image on the basis of the standard liver template. After that, if the patient's liver is more swollen or located at a lower place than the standard liver template, the correction unit 160 corrects the three-dimensional location of CV12 on the basis of the correction condition in advance set. For example, if the patient's CV12 coordinates X', Y' and Z' are within an area corresponding to the liver of the patient's three-dimensional image, the correction unit 160 previously sets conditions such as a location distant by distance 'a' from the surface of the liver of the patient or a location corresponding to percentage 'a' of a distance from the surface of the body of the patient to the surface of the liver of the patient and then corrects the coordinates to X'a, Y'a and Z'a, so that upon real acupuncture for CV12, the needle insertion into the patient's liver can be prevented. Like this, the three-dimensional acupoint mapping device 100 according to the present invention provides the individualized and customized acupuncture information based on the state and constitution of the patient.

The three-dimensional acupoint mapping device 100 according to the present invention utilizes the information on the three-dimensional acupoints and meridian pathways in the body as materials for education, and also provides really measured human body materials for the three-dimensional acupoints and meridian pathways in the body. Further, the three-dimensional acupoint mapping device 100 according to the present invention provides the information on the three-dimensional acupoint locations (the three-dimensional coordinates) on the body surface and in the deep body. Accordingly, the three-dimensional acupoint mapping device 100 provides the acupuncture information inclusive of the direction and depth of needle insertion.

Also, the three-dimensional acupoint mapping device 100 according to the present invention inputs the three-dimensional medical image information of the user (patient) to the standard templates to provide the user-customized acupoint location information. Accordingly, the present invention utilizes the three-dimensional coordinates of acupoints in the body as clinical assistant, thereby improving the accuracy and safety of the acupuncture.

Further, the three-dimensional coordinates of acupoints in the body determined in consideration of the states of the anatomical structures in the body, which are provided by the three-dimensional acupoint mapping device 100 according to the present invention, are used to analyze physiological and pathological states (that is, various biometric information inclusive of blood flow speed, the number of blood cells, ion concentration, and concentration of specific protein) in the deep portions of the acupoints as well as on the body surface of the acupoints, and are also used as traditional Korean medicine diagnostic information.

Also, the three-dimensional coordinates in the body for the acupoints provided by the three-dimensional acupoint mapping device 100 according to the present invention allow an artificial intelligence acupuncture device (or robot) to perform the acupuncture on the given acupoint more accurately and safely. Furthermore, a massage or acupressure device (or robot) for domestic use or commercial use can be developed with the information on the three-dimensional coordinates of acupoints and meridians in the body provided by the three-dimensional acupoint mapping device 100 according to the present invention

Further, the three-dimensional mapping device 100 according to the present invention is applicable to animals as well as human bodies.

### Mode for the Invention

On the basis of the above-mentioned three-dimensional mapping device 100, hereinafter, an explanation on a three-dimensional mapping method according to the present invention will be briefly given.

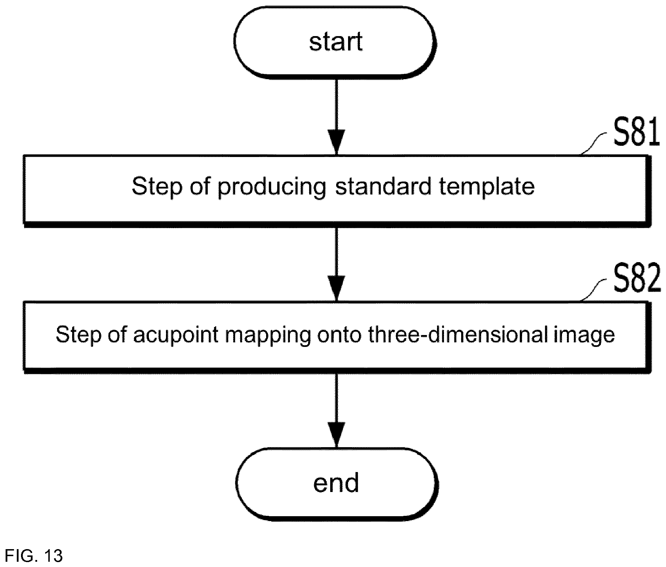

FIG.13 is a flowchart showing a three-dimensional acupoint mapping method according to the present invention.

As shown in FIG.13, the three-dimensional acupoint mapping method according to the present invention is carried out by means of the three-dimensional acupoint mapping device 100. Even if not explained, the contents explained on the three-dimensional acupoint mapping device 100 will be applied to the three-dimensional acupoint mapping method in the same manner.

Referring to FIG.13, a standard template is produced to determine a standard location of an acupoint on a three-dimensional standard image inclusive of at least a portion of the body at step S81. In this case, the standard location of the acupoint includes the three-dimensional coordinates in the body for the acupoint. Further, the standard location of the acupoint indicates the information on acupuncture inclusive of the direction and/or depth of needle insertion for the acupoint in consideration of the state of the anatomical structure on the three-dimensional standard image.

Further, the three-dimensional standard image is produced on the basis of the human body image inclusive of the information on the anatomical structures of the human body.

At step S82, next, the standard location of the acupoint on the three-dimensional standard image is determined on the basis of the produced standard template, and then, the determined standard location is mapped onto the three-dimensional standard image. At the step S82, at this time, the standard template for the anatomical structure on the three-dimensional standard image can be produced. At the step S82, further, the standard location can be determined in consideration of the state of at least one of the anatomical structures such as blood vessels, nerves, muscles, skeleton, and organs.

Even though not shown, on the other hand, the three-dimensional acupoint mapping method according to the present invention further includes the step of displaying three-dimensional meridian and acupoint information produced by mapping the standard location onto the three-dimensional standard image. At the displaying step, at this time, both of three-dimensional acupoint location information on the body surface and/or in the body and three-dimensional meridian pathway information are displayed.

If it is determined that a user's input for any acupoint is generated, at the displaying step, detailed acupuncture information on the acupoint corresponding to the user's input is displayed.

At this time, the detailed acupuncture information includes numerical information on the direction and/or depth of needle insertion into the acupoint from the body surface to the deep inside the body and information on the anatomical structure around the acupoint corresponding to the user's input.

At the displaying step, further, at least one operation of enlargement, reduction and rotation operations for the three-dimensional image is performed in response to the user's input related to image control. Through the operation, at this time, the information on the anatomical structure around the acupoint corresponding to the user's input is displayed according to the depths from the body surface to the deep body.

At the displaying step, furthermore, an emphasizing option menu for emphasizing at least one of the anatomical structures on the three-dimensional standard image is displayed. If the user's input for the emphasizing option menu is generated, at this time, the emphasizing option element corresponding to the user's input is identified, and the identified element is emphasized and displayed on the three-dimensional standard image.

Even though not shown, also, the three-dimensional acupoint mapping method according to the present invention further includes the step of, if a three-dimensional image of the user is obtained, identifying an anatomical structure from the user's three-dimensional image on the basis of the standard template.

Further, the three-dimensional acupoint mapping method according to the present invention further includes the step of, through the comparison between the user's three-dimensional image and the standard three-dimensional image, calculating a difference between the anatomical structure of the user's three-dimensional image and the anatomical structure of the standard three-dimensional image.

In addition, the three-dimensional acupoint mapping method according to the present invention further includes the step of correcting the standard location on the basis of the calculated difference between the two anatomical structures.

At the correction step, in this case, the standard location is corrected according to the state of the anatomical structure on the user's three-dimensional image on the basis of the difference between the states of the two anatomical structures. Through the correction, at this time, the user-customized three-dimensional acupoint information in the body is produced.

At the correction step, further, the correction on the standard location and the acupuncture information is performed in consideration of the type of the patient's disease and the acupuncture skill level of the practitioner.

The steps S81 and S82 may be segmented into a plurality of steps or combined to each other. Further, some of the steps may be removed if necessary, and the order in the steps may be changed.

On the other hand, a three-dimensional image displaying method according to another preferred embodiment of the present invention may be provided. In this case, the three-dimensional image displaying method according to the present invention is carried out by means of the three-dimensional acupoint mapping device 100. Even if not explained, the contents explained on the three-dimensional acupoint mapping device 100 will be applied to the three-dimensional image displaying method in the same manner.

Briefly, the three-dimensional image displaying method according to another preferred embodiment of the present invention includes the steps of (a) obtaining a three-dimensional image inclusive of at least a portion of the human body, (b) determining a location of at least one acupoint on the basis of the three-dimensional image, and (c) displaying the location of at least one acupoint in association with the three-dimensional image.

In this case, the three-dimensional image obtained at the step (a) is an image produced by reconstructing a medical human body image inclusive of the anatomical structure of the human body, which is obtained by CT, MRI, and so on. For example, the three-dimensional image obtained at the step (a) is a three-dimensional standard image or a three-dimensional image of a user.

At the step (b), further, three-dimensional coordinates on the body surface and/or in the body are determined as the location of acupoint.

At the step (b), at this time, the standard location of acupoint is determined in consideration of the state of at least one of the anatomical structures such as blood vessels, nerves, muscles, skeleton and organs on the three-dimensional image.

At the step (b), the determined location of acupoint is mapped onto the three-dimensional image, and at the step (c), after that, the three-dimensional image onto which the location of acupoint is mapped is displayed on a screen.

The three-dimensional acupoint mapping method according to the present invention is carried out in a form of program commands executed by various computer means and is recorded in computer readable media. The computer readable media include program commands, data files, and data structures solely or combinedly.

Further, the three-dimensional acupoint mapping method according to the present invention is carried out to the form of a computer program or application stored in the recording media and executed by the computer.

## Claims

1. A three-dimensional acupoint mapping method comprising the steps of:
(a) making use of a three-dimensional standard image inclusive of a three-dimensional image for an internal tissue of the human body to produce a standard template of the internal tissue for determining a standard location of an acupoint; and
(b) determining the standard location of the acupoint where a location of the internal tissue is considered on the three-dimensional standard image on the basis of the standard template of the internal tissue to map the determined standard location onto the three-dimensional standard image.

2. The three-dimensional acupoint mapping method according to claim 1, wherein the three-dimensional standard image is produced on the basis of a human body image inclusive of the information on the internal tissue of the human body, and at the step (a), the standard template of the internal tissue on the three-dimensional standard image is produced.

3. The three-dimensional acupoint mapping method according to claim 2, wherein at the step (b), the standard location of the acupoint is determined in consideration of the state of at least one of a blood vessel, nerve, muscle, skeleton and organ as the internal tissue.

4. The three-dimensional acupoint mapping method according to claim 2, wherein the standard location of acupoint comprises acupuncture information on direction and/or depth of needle insertion into the acupoint from the body surface to the deep body around which the state of the internal tissue is considered.

5. The three-dimensional acupoint mapping method according to claim 1, further comprising the step of (c) displaying the three-dimensional acupoint and meridian information produced by mapping the standard location onto the three-dimensional standard image.

6. The three-dimensional acupoint mapping method according to claim 1, further comprising the steps of:
(d) if a three-dimensional image of a user is obtained, identifying the internal tissue from the user's three-dimensional image on the basis of the standard template;
(e) through the comparison between the user's three-dimensional image and the standard three-dimensional image, calculating a difference between the internal tissue of the user's three-dimensional image and the internal tissue of the standard three-dimensional image; and
(f) correcting the standard location on the basis of the calculated difference between the two internal tissues.

7. The three-dimensional acupoint mapping method according to claim 6, wherein at the step (f), the standard location is corrected according to the state of the internal tissue on the user's three-dimensional image on the basis of the difference between the states of the two internal tissues, and through the correction, user-customized three-dimensional information on meridians and acupoints in the body is produced.

8. The three-dimensional acupoint mapping method according to claim 6, wherein at the step (f), the correction on the acupuncture information is performed in consideration of the state of the patient, the type of disease to be treated, the acupuncture skill level of a practitioner, and/or the precision of an acupuncture robot, and through the correction, user-customized acupuncture information is produced.

9. The three-dimensional acupoint mapping method according to claim 5, wherein at the step (c), if it is determined that the user's input for any one of the three-dimensional acupoint information is generated, detailed acupuncture information on the acupoint corresponding to the user's input is displayed, and the detailed acupuncture information comprises numerical information on the direction and/or depth of needle insertion into the acupoint from the body surface to the deep inside the body corresponding to the user's input and information on the internal tissue around the acupoint corresponding to the user's input.

10. The three-dimensional acupoint mapping method according to claim 5, wherein at the step (c), at least one operation of enlargement, reduction and rotation operations for the three-dimensional image is performed in response to the user's input related to image control, and through the operation, the information on the internal tissue around the acupoint corresponding to the user's input is displayed according to the depths from the body surface to the deep body.

11. The three-dimensional acupoint mapping method according to claim 5, wherein at the step (c), an emphasizing option menu for emphasizing at least one element of the internal tissues on the three-dimensional standard image is displayed, and if the user's input for the emphasizing option menu is generated, the emphasizing option element corresponding to the user's input is identified, so that the identified element is emphasized and displayed on the three-dimensional standard image.

12. The three-dimensional acupoint mapping method according to claim 5, wherein at the step (c), three-dimensional meridian pathway information on the body surface and/or in the body is further displayed.

13. A three-dimensional acupoint mapping device comprising:
a standard template producing unit for making use of a three-dimensional standard image inclusive of a three-dimensional image of an internal tissue of the human body to produce a standard template of the internal tissue for determining a standard location of an acupoint; and
a mapping unit for determining the standard location of the acupoint where a location of the internal tissue is considered on the three-dimensional standard image on the basis of the standard template of the internal tissue to map the determined standard location onto the three-dimensional standard image, wherein the standard location is three-dimensional coordinates in the body for the acupoint.

14. The three-dimensional acupoint mapping device according to claim 13, wherein the three-dimensional standard image is produced on the basis of a human body image inclusive of the information on the internal tissue of the human body, and the standard template producing unit produces the standard template of the internal tissue on the three-dimensional standard image.

15. The three-dimensional acupoint mapping device according to claim 14, wherein the mapping unit determines the standard location of the acupoint in consideration of the state of at least one of a blood vessel, nerve, muscle, skeleton and organ as the internal tissue.

16. The three-dimensional acupoint mapping device according to claim 14, wherein the standard location comprises acupuncture information on direction and/or depth of needle insertion into the acupoint from the body surface to the deep body around which the state of the internal tissue is considered.

17. The three-dimensional acupoint mapping device according to claim 13, further comprising a display controlling unit for displaying the three-dimensional information on meridians and acupoints produced by mapping the standard location onto the three-dimensional standard image.

18. The three-dimensional acupoint mapping device according to claim 13, further comprising:
an identification unit for identifying, if a three-dimensional image of a user is obtained, the internal tissue from the user's three-dimensional image on the basis of the standard template;
a calculation unit for calculating, through the comparison between the user's three-dimensional image and the standard three-dimensional image, a difference between the internal tissue of the user's three-dimensional image and the internal tissue of the standard three-dimensional image; and
a correction unit for correcting the standard location on the basis of the calculated difference between the two internal tissues.

19. The three-dimensional acupoint mapping device according to claim 18, wherein the correction unit corrects the standard location according to the user's input to the state of the patient, the disease to be treated, the acupuncture skill level of the practitioner, and/or the precision of an acupuncture robot, on the basis of the state of the internal tissue on the user's three-dimensional image, and through the correction, user-customized three-dimensional acupuncture information is produced.
